# EUROPÄISCHE PATENTSCHRIFT

(11) **EP 1 351 697 B1**
(45) Veröffentlichungstag und Bekanntmachung des Hinweises auf die Patenterteilung: **30.10.2013**
(21) Anmeldenummer: 02709881.3
(22) Anmeldetag: 17.01.2002
(51) Int. Cl.: A61P 19/00, A61K 38/18, A61K 38/19, A61K 38/36, A61K 38/39, A61K 38/48, A61K 33/00, A61K 33/24, A61K 33/42, A61K 35/14

(54) **ARZNEIMITTEL ZUR FÖRDERUNG DER REGENERATION VON GEWEBE**
PHARMACEUTICAL FOR STIMULATING THE REGENERATION OF TISSUES
AGENT PHARMACEUTIQUE DESTINE A FAVORISER LA REGENERATION DES TISSUS

(30) Priorität: 18.01.2001 AT 892001
(43) Veröffentlichungstag der Anmeldung: 15.10.2003
(73) Patentinhaber: Watzek, Georg, 1030 Wien (AT); Gruber, Reinhard, 2500 Baden (AT); Bio-Products & Bio-Engineering Aktiengesellschaft, 1010 Wien (AT)
(72) Erfinder: WATZEK, Georg, A-1030 Wien (AT); GRUBER, Reinhard, A-2500 Baden (AT); EIBL, Johann, A-1180 Wien (AT)
(74) Vertreter: Schwarz, Albin
(86) Internationale Anmeldenummer: PCT/AT2002/000018
(87) Internationale Veröffentlichungsnummer: WO 2002/056897

(56) Entgegenhaltungen:
- WO-A-00/71153
- WO-A-90/12581
- US-A- 5 165 938
- GEORGE J N ET AL: "PLATELET MEMBRANE MICROPARTICLES IN BLOOD BANK FRESH FROZEN PLASMA AND CRYOPRECIPITATE" BLOOD, Bd. 68, Nr. 1, 1986, Seiten 307-309, XP001095430 ISSN: 0006-4971

## Beschreibung

Die vorliegende Erfindung betrifft ein Arzneimittel zur lokalen Anwendung zur Förderung der Regeneration von Gewebe, insbesondere Knochengewebe.

Eukariontische Zellen können durch entsprechende Stimulation Teile ihrer Plasmamembran in den Extrazellularraum abgeben. Diese Zellfragmente enthalten zytoplasmatische Anteile und werden als Mikropartikel bezeichnet. Die Bildung derartiger Mikropartikel konnte bei Monozyten, bei Lymphozyten, Endothelzellen, Granulozyten und Thrombozyten nachgewiesen werden. Bei Thrombozyten kommt es zum Beispiel durch Stimulation mit Kollagen, Thrombin, dem Ca²⁺-Ionophor A23187 und dem Protein C5b-9 des Komplementsystems zur Abschnürung derartiger Zellelemente (Tans G., Blood 1991; Sims PJ., J Biol Chem 1988). Neben den genannten Substanzen, die eine Änderung der intrazellulären Kalziumkonzentration bewirken, sind auch Proteinphosphorylierungen, die Translokation von Phospholipiden, Änderungen im Zytoskelett und Scherkräfte an der Entstehung thrombozytärer Mikropartikel beschrieben.

Mikropartikel von Thrombozyten haben Eigenschaften, die sowohl zu einer Beschleunigung, als auch zu einer Verlangsamung der Blutgerinnung führen können. Durch die hochaffine Bindung der gerinnungsfördernden Faktoren VIII, einem Kofaktor des Tenaseenzymkomplexes und Va, der gemeinsam mit dem Faktor Xa den Prothrombinasekomplex ausbildet, kommt den Mikropartikeln eine koagulationsfördernde Funktion zu. Die Bindung der Faktoren an die Mikropartikel-Oberfläche erfolgt durch Phosphatidylserin, einem Phospholibid der Zellmembran. Umgekehrt führt die Anlagerung von "Protein S" zu einer Inaktivierung der Gerinnungsfaktoren Va und VIII, sowie zu einer Bindung der Proteine C und dem "aktivierten Protein C", woraus eine antikoagulative Eigenschaft der Mikropartikel abgeleitet werden kann (Tans G. Blood 1991). Verglichen mit aktivierten Thrombozyten weisen die Mikropartikel eine größere Anzahl an Bindungsstellen für die Gerinnungsfaktoren IXa (Hoffman, M., Thrombin Haemost 1992) und Va (Sims PJ., JBC 1988) auf. Die Gykoproteine GP IIb/IIIa, Ib, IaIIa und P-Selektin auf der Zelloberfläche ermöglichen darüber hinaus die Bindung der Mikropartikel an Gefäßendothelien (George JN, JCI 1986; Gawaz M, Aterioscler Thromb Vasc Biol 1996). Neben der Aktivierung von Endothelzellen konnte auch die Aktivierung von Monozyten und Thrombozyten durch Mikropartikel nachgewiesen werden (Barry OP; Thromb Haemat 1999).

Erhöhte Konzentrationen von Mikropartikeln in der Blutbahn wurden bei jenen Krankheiten beobachtet, die mit einer Aktivierung von Thrombozyten einhergehen.

Eine der wichtigsten Funktionen des Immunsystems ist, Leukozyten in die Nähe der Infektion und des verletzten Gewebes zu bringen. Dabei rollen die Leukozyten entlang der Endothelienwand und werden durch Integrine und Selektine zum Einwandern in das Wund-Infektionsareal veranlasst. Mikropartikel fördern die Akkumulation der "rollenden" Leukozyten durch P-Selektin und können dadurch zu einer verstärkten Hämostase und Entzündungsreaktion beitragen (Forlow B, Blood 2000). Die verstärkte Bindung von Monozyten an Endothelien durch Mikropartikel konnte ebenfalls nachgewiesen werden (Barry OP, JCI 1997). Eine weitere Arbeit konnte zeigen, dass Mikropartikel aus Thrombozyten zu einer vermehrten Proliferation von glatten Muskelzellen aus Gefäßen führen (Weber AA, Thromb Res 2000).

Thrombozyten sind die kleinsten Blutbestandteile im menschlichen Organismus und können auf chemische und physikalische Reize reagieren. Im Falle einer Gefäßverletzung werden Thrombozyten veranlaßt, an freigelegten endothelialen Oberflächen zu aggregieren und eine Vielzahl von biologisch aktiven Substanzen zu sezernieren. Dazu zählen Platelet-derived growth factor (PDGF), Transforming growth factor-β (TGF-β), Epidermal growth factor (EGF), aber auch Metaboliten der Arachidonsäure, wie Prostaglandin D₂/E₂ und Thromboxan A₂, und auch Mikropartikel.

Es ist anzunehmen, dass durch die Freisetzung der Mikropartikel die Ausbildung des Fibrinclots und das Einwandern von Entzündungszellen in das Wundareal gefördert wird. Die neutrophilen Granulozyten und Makrophagen sezernieren ihrerseits Wachstumsfaktoren, die wiederum Leukozyten, Fibroblasten und Endothelzellen in den Fibrinclot führen. Makrophagen bauen zerstörtes Gewebe ab und fördern die Proliferation und die Synthese von Kollagen Typ I, wodurch die Ausbildung des sogenannten Grantulationsgewebes eingeleitet wird. Dabei handelt es sich um ein fibröses Bindegewebe, welches das ursprüngliche Gewebe ersetzt. Parallel dazu kommt es zur Einsprossung von Gefäßen und zur Epithelialisierung des Wundareals. Die Entstehung eines zellarmen, kollagenreichen dauerhaften Narbengewebes bildet den Abschluss der Wundheilung, ein Prozess der Wochen aber auch Monate dauern kann.

Da ein Narbengewebe nicht die ursprünglichen Eigenschaften aufweist, spricht man bei der Heilung von Weichgewebe von Reparation, nicht aber von Regeneration. (Bennet NT et al. Am. J. Surg. 1993, 165: 728-737; Bennet NT et al. AM. J. Surg. 1993, 166: 74-81).

Es gibt jedoch Störungen der Wundheilung, die durch zahlreiche Ursachen hervorgerufen werden können. Hyperglykämie behindert die Wundheilung möglicherweise über eine Hemmung der Proliferation von Endothelzellen und Fibroblasten (Goodson WH, J Surg Res 1977 22: 221-227). Erhöhte Blutzuckerwerte vermindern außerdem die Leukozytenfunktion, wodurch die Wunde in der Entzündungsphase verweilt. Weiters kann auch der Schweregrad des vorangegangenen Traumas Anlaß für einen ungünstigen Ablauf der Wundheilung sein (Holzheimer RG, 1966, Zentralbl Chir 121:231).

Im Gegensatz zur Ausbildung eines Narbengewebes bei Weichteilverletzungen (Reparation) kommt es nach Knochenbrüchen bzw. nach Transplantation von Knochen zur vollständigen Wiederherstellung der ursprünglichen Gewebestruktur (Regeneration). Prinzipiell ähneln die regenerativen Vorgänge des Knochens dem Heilungsschema der Weichteilwundheilung: Unmittelbar nach der Verletzung kommt es u.a. zur Freisetzung von PDGF und TGF-β durch die degranulierenden Thrombozyten, gefolgt von der Einwanderung von Makrophagen und anderen Entzündungszellen, die ebenfalls PDGF, TGF-β, aber auch Fibroblast growth factor (FGF), Interleukin-1 (IL-1) und IL-6 sezemieren. Es wird generell angenommen, daß dieses komplexe Zusammenwirken von Wachstumsfaktoren gemeinsam mit dem sich ausbildenden Fibringerüst den initialen Prozeß der Knochenheilung darstellt, wobei die umliegenden Gewebe, wie Knochenmark, Periost und Weichteile, maßgeblich an der Regeneration beteiligt sind.

Neben der Reorganisation der Knochenmarkzellen in Zonen unterschiedlicher Dichte werden die den Knochen auskleidenden Osteoblasten und die Preosteoblasten des Kambiums zur Zellteilung und Differenzierung angeregt. Der durch diese Vorgänge neugebildete Geflechtknochen wird als harter Kallus bezeichnet. Neben der direkten Ossifikation kommt es zur Einwanderung von undifferenzierten mesenchymalen Zellen aus dem Periost und von Fibroblasten aus dem umgebenden Weichgewebe in das Hämatom. Nach einer extensiven Teilungsphase entsteht ein knorpeliges Gewebe, der weiche Kallus, dessen Zellen hypertrophieren, mineralisieren und nach der Einsprossung von Gefäßen durch Geflechtknochen ersetzt werden. Der letzte Schritt zur vollständigen Wiederherstellung der ursprünglichen Knochenstruktur ist der Umbau des Geflechtknochens zu lamellärem Knochen durch Osteoblasten/Osteoblastentätigkeit. Dieser Vorgang wird als indirekte Ossifikation bezeichnet, da der entstandene Knorpel erst durch Knochen ersetzt werden muß (Barnes et al., JBMR 1999, 11:1805-1815).

Die Knochenbruchheilung verläuft nicht immer problemlos: Infektionen, Systemerkrankungen (z.B. Osteoporose), Stoffwechselerkrankungen (z.B. Diabetes mellitus), genetische Defekte (z.B. Osteogenesis imperfecta) und medikamentöse Behandlungen (Glucocorticoidtherapie) können Ursache einer verzögerten Regeneration sein.

Neben der Frakturheilung kommt der Transplantation von autologem Knochen und Knochenersatzmaterial für augmentative Verfahren oder zur Auffüllung von Knochendefekten eine zunehmende Bedeutung zu. Auch in diesem Fall wäre eine Beschleunigung der Regeneration und eine Verbesserung der "Knochenqualität" wünschenswert.

Die genauen Ursachen von verlangsamter oder ausbleibender Knochenregeneration sind nicht bekannt. Aus der WO 91/13905, der WO 91/04035, der WO 91/16009, der US-A-5,165,938 und der US-A - 5,178,883 ist bekannt, dass aus Thrombozyten freigesetzte Wachstumsfaktoren zur Wundheilung eingesetzt werden können.

Die Aktivierung von Thrombozyten ist z.B. aus der WO 86/03122 bekannt. Bei der Aktivierung werden Wachstumsfaktoren für Fibroblasten und Muskelzellen abgeben. Das durch Aktivierung erhaltene Produkt kann mit einem Träger, z.B. mikrokristallinem Kollagen, zu einer Salbe verarbeitet werden.

Gemäß der WO 90/07931 kann diese Salbe auch zur Unterstützung von Haarwuchs eingesetzt werden.

Die WO 00/15248 beschreibt eine Zusammensetzung, welche thrombozytäre Wachstumsfaktoren enthält und Fibrin und ein weiteres Polymer enthält. Diese Zusammensetzung kann zur Heilung und Behandlung von Schäden in Geweben verwendet werden, die durch geringe Durchblutung und/oder veminderte Regenerationsfähigkeit charakterisiert sind, wobei zu diesen Geweben insbesondere elastische bzw. hyaline FaserKnorpel und Fasziengewebe gehören.

Der Gelenksknorpel ist ein avaskuläres Gewebe mit einem limitierten Regenerationspotential. Keine der derzeit in Verwendung stehenden Methoden zur Erneuerung von Gelenksknorpel von Patienten mit Osteoarthrose kann als zufriedenstellend angesehen werden. Derzeit ist eine Methode zugelassen, bei der autologe Knorpelzellen ex vivo expandiert und dann unter einen Periostlappen gebracht in den Defekt eingebracht werden (Brittenberg M, NEJM 1994).

Die vorliegende Erfindung stellt sich die Aufgabe, ein Arzneimittel mit verbesserter Wirkung bei der Regeneration von Gewebe, insbesondere Knochengewebe zur Verfügung zu stellen.

Das erfindungsgemäße Arzneimittel ist dadurch gekennzeichnet, dass es
- Mikropartikel enthält, die aus Thrombozyten stammen und durch eine aktivierende Behandlung der Thrombozyten mit Thrombin, Kollagen, dem Ca2+-Ionophor A23187 und/oder dem Protein C5b-9 in wässeriger Lösung erhältlich sind und mittels Differentialzentrifugation, Filtration oder Affinitätschromatographie gereinigt sind,
- einem Verfahren zur Virusinaktivierung und/oder Virusabreicherung unterzogen ist,
- und daß durch Sterilfiltration Keimfreiheit erreicht wurde, und daß es
- in lyophilisiertem oder tiefgefrorenem Zustand vorliegt.

Die Erfindung beruht auf der Erkenntnis, dass die aus den eukariontischen Zellen freigesetzten Mikropartikel die Proliferation von Fibroblasten, osteoblastären und Knorpelzellen stimulieren, d.h. fördern. Die Mikropartikel können dabei homologen Ursprungs sein. Unter dem Ausdruck Mikropartikel sind all jene Zellbestandteile zu verstehen, welche aus einer wässrigen Suspension unter den aus der Literatur bekannten Methoden (z.B.: Zentrifugation bei 100 000 x g / 2h; Forlow SB, Blood 2000) abgetrennt werden können. Die abzentrifugierten Mikropartikel können einem Verfahren zur Virusabweichung und/oder Virusinaktivierung unterzogen werden. Zweckmäßig kann das Arzneimittel mit Wachstumsfaktoren versehen sein.

Das erfindungsgemäße Arzneimittel kann hergestellt werden, indem Thrombozyten in einem wässerigen Medium einer aktivierenden Behandlung unterzogen werden, um die regenerationsfördernden Mikropartikel aus den Thrombozyten freizusetzen, wonach das wässerige Medium, welches die freigesetzten Mikropartikel enthält, zum Sedimentieren der groben Zellbestandteile zentrifugiert wird. Die partikulären Bestandteile des erhaltenen wässrigen Überstandes werden in einem 2. Zentrifugationsschritt bei hoher g-Zahl (z.B. 100 000 x g) gewonnen und einem Verfahren zur Virusabreicherung und/oder Virusinaktivierung unterzogen. Beispiele für eine aktivierende Behandlung ist das Kontaktieren der Thrombozyten mit Thrombin, Kollagen, dem Ca²⁺-Ionophor A23187 und/oder Protein C5b-9 des Komplementsystems. Das erfindungsgemäße Arzneimittel liegt in tiefgefrorenem oder lyophylisiertem Zustand vor.

Das erfindungsgemäße Arzneimittel lässt sich auch wiederholt applizieren, wodurch die folglich höhere Konzentration der Mikropartikel im Wundareal eine raschere Ausbildung von Granulationsgewebe ermöglicht. Gleichzeitig kann eine provisorische extrazelluläre Matrix aus organischen (z.B. Fibrin, Kollagen, Polyaktonen etc.) oder aus anorganischen Materialien (Kalziumphosphate etc.) verabreicht werden, die als Trägersubstanz für die Wachstumsfaktoren und als Gerüst für die einwandernden Zellen dient.

Die kovalente Bindung des erfindungsgemäßen Arzneimittels an die oben genannten Matrices kann durch Transglutaminasen erfolgen.

Es besteht weiters die Möglichkeit, Metalloberflächen mit dem erfindungsgemäßen Arzneimittel zu versehen.

Zur Virusabreicherung und/oder Virusinaktivierung eignen sich im Stand der Technik bekannte physikalische, chemische oder physikalisch/chemische Kombinationsverfahren.

Die Keimfreiheit des erfindungsgemäßen Arzneimittels wird entweder durch sterile Gewinnung der Zellkonzentrate und aseptische Weiterverarbeitung, oder durch Sterilfiltration erreicht.

Die Gewinnung der Mikropartikel, die Herstellung des erfindungsgemäßen Arzneimittels und dessen Wirkung auf osteoblastäre Zellen wird im folgenden beispielhaft näher erläutert.

### Gewinnung der Mikropartikel

Ein Thrombozytenkonzentrat (2x10⁹ Zellen) wird mit einem Überschuß von Thyrode-Puffer (pH=6,4) gemischt und 10 min bei 1200 x g zentrifugiert. Der Überstand wird abgegossen, das Thrombozyten-Pellet in 2 ml DMEM/F12-ITS resuspendiert und mit 10 µM Ca²⁺-Ionophor A23187 (Sigma) 30 min bei Raumtemperatur inkubiert. Durch diese Behandlung werden Mikropartikel aus den Thrombozyten freigesetzt.

Anschließend wird neuerlich 10 min bei 1200 x g zentrifugiert, wobei sich ein Niederschlag und ein Überstand bilden. Der Überstand (= der Thrombozytenüberstand), der die aus den Thrombozyten freigesetzten Mikropartikel enthält, wird abgehoben und einer weiteren Zentrifugierung unterworfen.

Dabei werden die durch die Aktivierung der Thrombozyten freigesetzten Mikropartikel 1 h bei 14 000 x g, 4°C abzentrifugiert und das entstandene Pellet (=Mikropartikelpellet) in 2 ml DMEM/F12-IST resuspendiert.

Zur Gewinnung der Mikropartikel können statt des oben beschriebenen Ca²⁺-Ionophores A23187 auch Thrombin (Baxter, Österreich) oder andere, oben beschriebene Agentien, verwendet werden.

### Virusinaktivierung des Thrombozytenüberstandes (photodynamische Virusinaktivierung)

Zu 50 ml einer gemäß obigem Verfahren hergestellten Mikropartikel-Suspension wird 8-Methoxypsoralen (gelöst in Dimethylsulfoxid [DMSO]) bis zu einer Endkonzentration von 300µg/ml (Endkonzentration von DMSO 0,3 %) zugesetzt und bei 22-27°C unter einer Atmosphäre von 5 % CO₂ und 95 % N₂ und einem Druck von 2 psi sechs Stunden lang mit ultraviolettem Licht von unten und oben bestrahlt, so daß die gesamte Lichtintensität 3,5 bis 4,8 mW/cm² beträgt (Lin L. et al. Blood 1989). Auf diese Weise wird die Mikropartikel-Suspension virusinaktiviert.

Nach durchgeführter Virusinaktivierung kann die Suspension tiefgefroren oder lyophilisiert werden, wie nachfolgend beschrieben wird.

Tieffrierung: Je 1 ml der Mikropartikelsuspension wird bei -80°C innerhalb von 30-40 Minuten schock-tiefgefroren gelagert. Vor der Verwendung wird das Präparat bei Zimmertemperatur aufgetaut.

Lyophilisierung: Je 1 ml des wird im Mikropartikelsuspension bei -80°C für mindestens 24 Stunden tiefgefroren und anschließend bei -20°C bis -40°C über 20 bis 24 Stunden unter Vakuum gefriergetrocknet. Die gefriergetrockneten Überstände werden zwischen -20°C und -80°C gelagert und vor Verwendung mit DMEM/F12-Medium rehydriert.

### Virusinaktivierung einer provisorischen extrazellulären Matrix, die Gerüstsubstanzen enthält (chemische Virusinaktivierung)

Matrices, die einer gemäß obigem Verfahren hergestellten Mikropartikel-Suspension zugesetzt werden, werden durch die Solvent-Detergent-Methode virusinaktiviert. Dazu wird einer Suspension der Matrices bei 30°C 1 % (Gew./Gew.) Tri(n-butyl)phosphat und 1 % (Gew./Gew.) Triton X-100 zugesetzt und das Gemisch vier Stunden geschüttelt. Danach wird unter Zusatz von 5 % (Vol./Vol.) Sojabohnenöl die Solvent-Detergent-Mischung aus der Suspension der Biomaterialien an einer C18-Säule (Waters, Millipore) durch Chromatographie entfernt (Horowitz B. et al., Blood 1992, 79-826-831; Piet MP. et al., Transfusion 1990, 30:591-598; Piquet Y. et al., 1992, 63:251-256).

Die mit der oben beschriebenen chemischen Virusinaktivierungsmethode behandelten Matrices können nachfolgend zusätzlich noch einer photodynamischen Virusinaktivierung unterzogen werden.

### Kultivierung von humanen Osteoblasten

Die Gewinnung primärer humaner Osteoblasten kann aus Knochenstücken mit einer Größe von etwa 1-5 mm³ erfolgen. Dazu werden die Knochenstücke mit Phosphat-gepufferter Salzlösung (PBS) gewaschen und 2-3 Wochen bei 37°C, 95% Luftfeuchtigkeit und 5% CO₂ kultiviert. Als Kulturmedium wird DMEM/F12 unter Zusatz von 10% fötalem Kälberserum (FBS), Antibiotika und Fungiziden verwendet.

Die aus den Knochenstücken auswachsenden Osteoblasten werden mit Trypsin (2,5%) von den Zellkulturflaschen abgelöst und nach einem 1:3 Verdünnungsschritt unter denselben Bedingungen weitergezogen (Passage 1). Dieser Vorgang wird zur Vermehrung der Zellen zweimal wiederholt. Medien und Zusätze können von Life Technologies, Grand Island, NY, USA, bezogen werden.

Zur anschließenden Stimulierung der Osteoblastenproliferation mit den aus den Thrombozyten zu gewinnenden Mikropartikeln werden die Osteoblasten mit einer Dichte von 10.000 Zellen/cm² in Mikrotiterplatten (Packard, Meriden, CT, USA) angesetzt und 2-4 Tage in Vollmedium vorkultiviert, welches zu Testzwecken durch ein serumfreies Medium ersetzt wird. Dabei handelt es sich um ein DMEM/F12-Medium, bei dem anstelle von FBS eine Mischung aus 5 mg/ml Insulin/Transferrin/Selen (ITS, Boehringer Mannheim, DE) zugesetzt wird.

### Kultivierung von humanen Fibroblasten

Die Gewinnung primärer humaner Fibroblasten kann aus Stücken von Mundschleimhaut erfolgen. Dazu werden die Mundschleimhautstücke mit PBS gewaschen und 2-3 Wochen bei 37°C, 95% Luftfeuchtigkeit und 5% CO₂ kultiviert. Als Zellkulturmedium wurde DMEM/F12 unter Zusatz von 10% FBS, Antibiotika und Fungizigen verwendet. Die aus den Mundschleimhautstücken auswachsenden Fibroblasten (Gingivafibroblasten) wurden mit Trypsin (2,5%) von den Zellkulturflaschen abgelöst und nach einem 1:3 Verdünnungsschritt unter den selben Bedingungen weitergezogen (Passage 1). Dieser Vorgang wurde zur Vermehrung der Zellen zweimal wiederholt. Medien und Zusätze können von Life Technologies bezogen werden (Gomstein RA, J Periodontol 1999).

### Kultivierung von humanen Chondrozyten

Die Gewinnung primärer humaner Chondrozyten kann aus Stücken von Gelenksknorpel erfolgen. Dazu werden die Knorpelstücke mit PBS gewaschen, zerkleinert und die Zellen durch Verdauung mit einer Kollagenase B Lösung (0,4% Gew./Vol.; Boehringer Mannheim, Deutschland) freigesetzt. Die weiteren Schritte sind oben beschrieben (F Héraud, Ann Rheum Dis 2000).

### Miotische Aktivität der Mikropartikel

Die gemäß obigem Verfahren erhaltenen Mikropartikel-Präparationen wurden auf deren miotische Aktivität untersucht.

Zur Bestimmung der biologischen Aktivität wurden die Präparationen im Verhältnis 1:5 in DMEM/F12-IST verdünnt. Die so erhaltene Verdünnung wird als Erstverdünnung (I) bezeichnet und entspricht einer Mikropartikel-Konzentration, die aus einer ursprünglichen Thrombozytenzahl von 2x10⁸ Zellen/ml gewonnen wurde.

Aus einem Teil der Erstverdünnung (I) wird durch weiteres Verdünnen im Verhältnis von 1:5 eine Verdünnungsreihe aufgestellt, welche den Überständen von 4x10⁷ Zellen/ml (Zweitverdünnung II), 8x10⁶ Zellen/ml (Drittverdünnung III), 1,6x10⁶ Zellen/ml (Viertverdünnung (IV) und 3,2x10⁵ Zellen/ml (Fünftverdünnung V) entsprechen.

### Stimulierung der Osteoblastenproliferation

Mit den erhaltenen 5 Verdünnungsstufen I, II, III, IV und V wird die Osteoblastenproliferation wie folgt stimuliert:

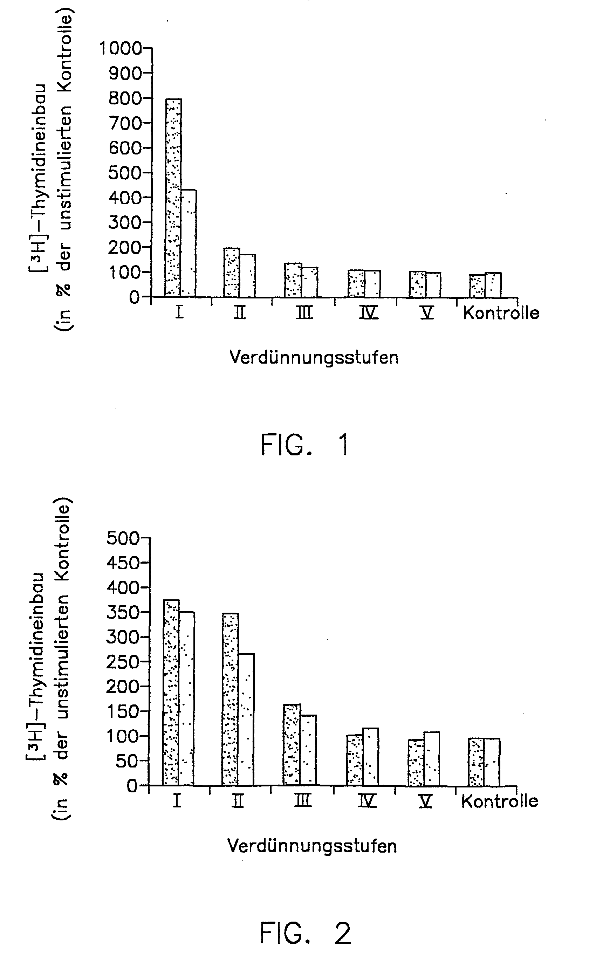

4x100 µl jeder Verdünnungsstufe werden 24h mit Osteoblasten kultiviert. Während der letzten 6 Stunden erfolgt die Zugabe von 1 µCi [³H]-Thymidin/Tüpfel, dessen Einbaurate als Maß für die Osteoblastenproliferation genommen wird. Die aufgenommene Radioaktivität wird durch Flüssigkeitsszintillation (Packard) bestimmt. Als Kontrolle dient DMEM/F12-ITS, wobei der damit erhaltene Wert als 100% angenommen wird. Die Figur 1 zeigt die mit den Verdünnungsstufen I-V und mit der Kontrolle erzielte Osteoblastenproliferation.

Figur 1 dokumentiert eine dosisabhängige Osteoblastenproliferation, wobei in der höchsten Konzentration (Verdünnungsstufe I) ca. 3-7 mal mehr [³H]-Thymidin in die DNA eingebaut wird als in der Kontrolle ohne Mikropartikel.

### Stimulierung der Fibroblastenproliferation

Mit den erhaltenen 5 Verdünnungsstufen I, II, III, IV und V werden die Fibroblasten wie oben beschrieben stimuliert:

Fig. 2 dokumentiert eine dosisabhängige Zellproliferation, wobei in der höchsten Konzentration (Verdünnungsstufe I) ca. 2-3 mal mehr [³H]-Thymidin in die DNA eingebaut wird als in der Kontrolle ohne Mikropartikel (Schwarzer Balken: Mikropartikel von Spender A, Weiße Balken: Mikropartikel von Spender B).

### Stimulierung der Chondrozytenproliferation

Mit den erhaltenen 5 Verdünnungsstufen I, II, III, IV und V werden die Chondrozyten wie oben beschrieben stimuliert:

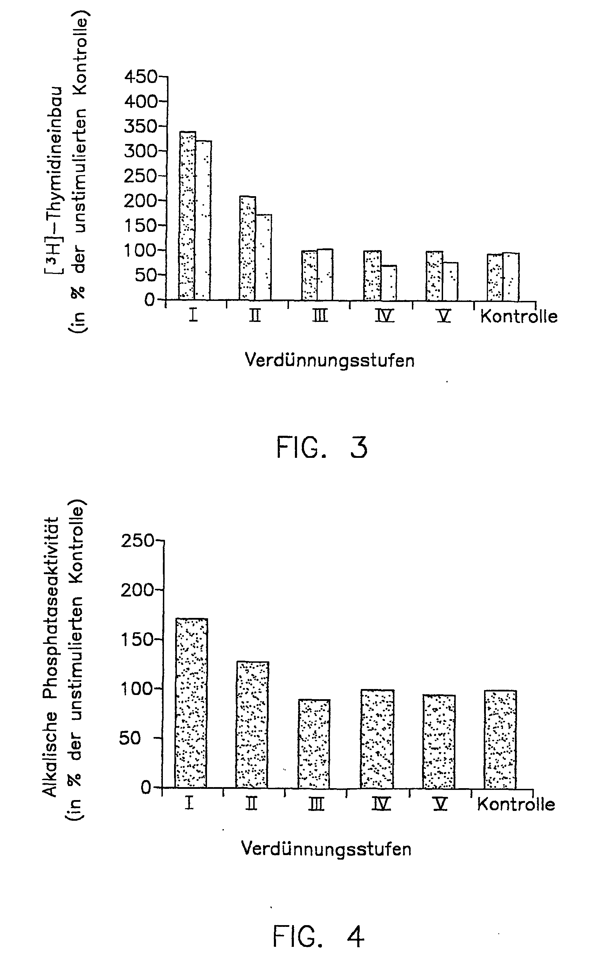

Fig. 3 dokumentiert eine dosisabhängige Zellproliferation, wobei in der höchsten Konzentration (Verdünnungsstufe I) ca. 2-3 mal mehr [³H]-Thymidin in die DNA eingebaut wird als in der Kontrolle ohne Mikropartikel (Schwarzer Balken: Mikropartikel von Spender A, Weißer Balken: Mikropartikel von Spender B).

### Stimulierung der Differenzierung der osteoblastären Zellen

Mit den erhaltenen 5 Verdünnungsstufen I, II, III, IV und V werden die osteoblastären Zellen wie folgt stimuliert:

4x 100µl jeder Verdünnungsstufe werden für 4 Tage mit den osteoblastären Zelle kultiviert. Danach werden die Zellen mit PBS gewaschen und in 100µl einer 0,5% Triton-X100 Lösung lysiert. Aus dem Lysat werden jeweils 20µl für die Bestimmung des Gesamtproteins verwendet (Gruber R, Cytokine 2000). Die gemessene Enzymaktivität wird auf die Proteinmenge normiert. Diese Methode dient zur Bestimmung der Differenzierung von Osteoblasten. Als Kontrolle wird DMEM/F12-ITS verwendet, wobei der damit erhaltene Wert als 100% angenommen wird.

Fig. 4 zeigt eine Stimulation der Differenzierung der osteoblastären Zellen in der Verdünnungsstufe I. Die Aktivität der alkalischen Phosphate ist ca. 80% höher als in deren Vergleichsgruppe ohne Mikropartikel.

### Bindung der Mikropartikel an eine provisorische extrazelluläre Matrix, die Gerüstsubstanzen enthält

Der gemäß obigem Verfahren hergestellten sterilen und virusinaktivierten Mikropartikelsuspension wird eine Lösung einer provisorischen extrazellulären Matrix, die Gerüstsubstanzen enthält, zugesetzt. Dabei handelt es sich sowohl um vernetzbare Biomaterialien (Fibrinogen, Fibronectin, Blutgerinnungsfaktor XIII, Kollagen), die einem oder mehreren Verfahren zur Virusinaktivierung unterzogen worden sein können, oder auch um organische (z.B.: Polylaktone) und anorganische Materialien (z..B.: Kalziumphosphate). Die Komponenten können einzeln oder in Kombination miteinander zur Anwendung gelangen. Das Mischungsverhältnis der Mikropartikelsuspension mit der extrazellulären Matrix soll vorzugsweise 1:3 betragen. Die so erhaltene Mischung wird zur Erzielung einer zweckmäßigen Haltbarkeit gemäß mit dem oben dargestellten Verfahren tiefgefroren oder lyophilisiert.

Anstelle der beschriebenen Durchführung der Virusinaktivierung an den Einzelkomponenten ist es auch möglich, die Virusinaktivierung an dem Gemisch von Mikropartikelsuspension und zugesetzter Matrix vorzunehmen.

Weiters besteht die Möglichkeit, die Mikropartikel an die oben genannten Matrices durch kovalente Bindung mittels Transglutaminasen zu binden.

## Patentansprüche

1. Arzneimittel zur lokalen Anwendung zur Förderung der Geweberegeneration, **dadurch gekennzeichnet, dass** es
- Mikropartikel enthält, die aus Thrombozyten stammen und durch eine aktivierende Behandlung der Thrombozyten mit Thrombin, Kollagen, dem Ca2+-Ionophor A23187 und/oder dem Protein C5b-9 in wässeriger Lösung erhältlich sind und mittels Differentialzentrifugation, Filtration oder Affinitätschromatographie gereinigt sind,
- einem Verfahren zur Virusinaktivierung und/oder Virusabreicherung unterzogen ist,
- und daß durch Sterilfiltration Keimfreiheit erreicht wurde, und daß es
- in lyophilisiertem oder tiefgefrorenem Zustand vorliegt.

2. Arzneimittel nach Anspruch 1, **dadurch gekennzeichnet, daß** lösliche oder unlösliche wundheilungsfördernde Substanzen enthalten sind.

3. Arzneimittel nach Anspruch 1 oder Anspruch 2, **dadurch gekennzeichnet, daß** Zytokine und/oder Wachstumsfaktoren enthalten sind.

4. Arzneimittel nach einem der Ansprüche 1 bis 3, **dadurch gekennzeichnet, daß** es eine Substanz enthält, die eine provisorische, extrazelluläre Matrix darstellt oder ausbilden kann.

5. Arzneimittel nach einem der Ansprüche 1 bis 4, **dadurch gekennzeichnet, daß** es Kollagen enthält.

6. Arzneimittel nach einem der Ansprüche 1 bis 5, **dadurch gekennzeichnet, daß** es zur Bildung eines Fibringerüstes Fibrinogen und Thrombin enthält.

7. Arzneimittel nach Anspruch 4, **dadurch gekennzeichnet, daß** als provisorische, extrazelluläre Matrix ein organisches Polymer, insbesondere ein Polylacton, vorgesehen ist.

8. Arzneimittel nach einem der Ansprüche 1 bis 7, **dadurch gekennzeichnet, daß** es anorganische Verbindungen enthält.

9. Pharmazeutisches Produkt, **dadurch gekennzeichnet, daß** es aufweist:
- ein Arzneimittel gemäß einem der Ansprüche 1 bis 8 und
- ein biokompatibles Material, welches zusammen mit dem Arzneimittel angewendet wird.

## Claims

1. A drug composition to be applied topically for promoting the regeneration of tissue, **characterized in that**
- it contains microparticles from thrombocytes which are obtainable in aqueous solution by activating treatment of the thrombocytes with thrombin, collagen, the Ca²⁺ ionophor A23187 and/or the protein C5b-9, and which have been purified by differential centrifugation, filtration or affinity chromatography,
- it has been subjected to a procedure for virus inactivation and/or virus depletion,
- and that sterility was achieved by sterile filtration, and
- it is provided in freeze-dried or deep-frozen state.

2. A drug composition according to claim 1, **characterized in that** it contains soluble or insoluble substances promoting wound healing.

3. A drug composition according to any one of claims 1 or 2, **characterized in that** it contains cytokines and/or growth factors.

4. A drug composition according to any one of claims 1 to 3, **characterized in that** it contains a substance which constitutes or may form a provisional extracellular matrix.

5. A drug composition according to any one of claims 1 to 4, **characterized in that** it contains collagen.

6. A drug composition according to any one of claims 1 to 5, **characterized in that** it contains fibrinogen and thrombin for the formation of a fibrin scaffold.

7. A drug composition according to claim 4, **characterized in that** an organic polymer, in particular a polylacton, is provided as the provisional extracellular matrix.

8. A drug composition according to any one of claims 1 to 7, **characterized in that** it contains inorganic compounds.

9. A drug product **characterized in that** it contains:
- a drug composition according to any of claims 1 to 8, and
- a biocompatible material which is applied together with the drug composition.

## Revendications

1. Agent pharmaceutique pour un emploi local destiné à la régénération des tissus, **caractérisé en ce**
- **qu'**il contient des microparticules qui proviennent de thrombocytes et qui peuvent être obtenues par un traitement d'activation des thrombocytes avec de la thrombine, du collagène, de l'Ionophor A23187- Ca2+ et/ou de la protéine C5b-9 en solution aqueuse, et sont purifiées au moyen d'une centrifugation différentielle, d'une filtration ou d'une chromatographie d'affinité,
- **qu'**il est soumis à un procédé destiné à l'inactivation des virus et/ou à l'appauvrissement en virus,
- et **qu'**une exemption de germes est réalisée par une filtration stérile, et qu'il
- est présent à l'état lyophilisé ou congelé.

2. Agent pharmaceutique selon la revendication 1, **caractérisé en ce que** des substances solubles ou insolubles favorisant la guérison des plaies sont incluses.

3. Agent pharmaceutique selon la revendication 1 ou la revendication 2 **caractérisé en ce que** des cytokines et/ou des facteurs de croissance sont inclus.

4. Agent pharmaceutique selon l'une des revendications 1 à 3 **caractérisé en ce qu'**il contient une substance qui représente, ou peut former, une matrice provisoire, extracellulaire.

5. Agent pharmaceutique selon l'une des revendications de 1 à 4 **caractérisé en ce qu'**il contient du collagène.

6. Agent pharmaceutique selon l'une des revendications de 1 à 5 **caractérisé en ce qu'**il contient du fibrinogène et de la thrombine pour la formation d'un échafaudage de fibrine.

7. Agent pharmaceutique selon la revendication 4 **caractérisé en ce qu'**un polymère organique, en particulier, une polylactone, est prévu en tant que matrice provisoire, extracellulaire.

8. Agent pharmaceutique selon l'une des revendications 1 à 7, **caractérisée en ce qu'**il contient des composés inorganiques.

9. Produit pharmaceutique **caractérisé en ce qu'**il présente :
- un agent pharmaceutique selon l'une des revendications de 1 à 8, et
- un matériau biocompatible qui est employé conjointement avec l'agent pharmaceutique.
